# DEMANDE DE BREVET EUROPEEN

(11) **EP 4 640 134 A1**
(43) Date de publication de la demande: **29.10.2025**
(21) Numéro de dépôt: 25171201.4
(22) Date de dépôt: 17.04.2025
(51) Int. Cl.: A61B 3/08

(54) **DÉTERMINATION D'UNE VALEUR DE RÉSERVES FUSIONNELLES D'UN SUJET**

(30) Priorité: 24.04.2024 FR 2404237
(71) Demandeur: SiVIEW, 75014 Paris (FR)
(72) Inventeur: ENGEL, Léa, 75015 Paris (FR); COLLOBERT, Guylène, 75011 Paris (FR); PICHEREAU, Laure, 91460 MARCOUSSIS (FR)
(74) Mandataire: Cabinet Camus Lebkiri

(57) **Abrégé**

L'invention concerne un procédé de détermination d'au moins une valeur de réserves fusionnelles d'un sujet, le procédé étant mis en œuvre par un module de commande (900) configuré pour commander une tête de réfracteur automatique (920).

L'invention concerne également un dispositif (910) et un système de détermination d'au moins une valeur de réserves fusionnelles d'un sujet et un produit programme d'ordinateur associé.

## Description

### DOMAINE TECHNIQUE

La présente invention concerne de manière générale le domaine de l'optométrie.

L'invention concerne plus particulièrement un procédé, un dispositif et un système pour déterminer, de manière automatique, au moins une valeur de réserves fusionnelles d'un sujet.

### ÉTAT DE LA TECHNIQUE

L'hétérophorie correspond à une déviation des axes visuels lorsqu'un des deux yeux ou les deux yeux sont au repos. Elle n'est mise en évidence que lorsque la vision des deux yeux est dissociée. En effet, lorsque la vision des deux yeux n'est pas dissociée (vision binoculaire), en présence d'une vision binoculaire normale, le cerveau superpose et fusionne les images reçues (une pour chaque œil) afin d'obtenir une seule image.

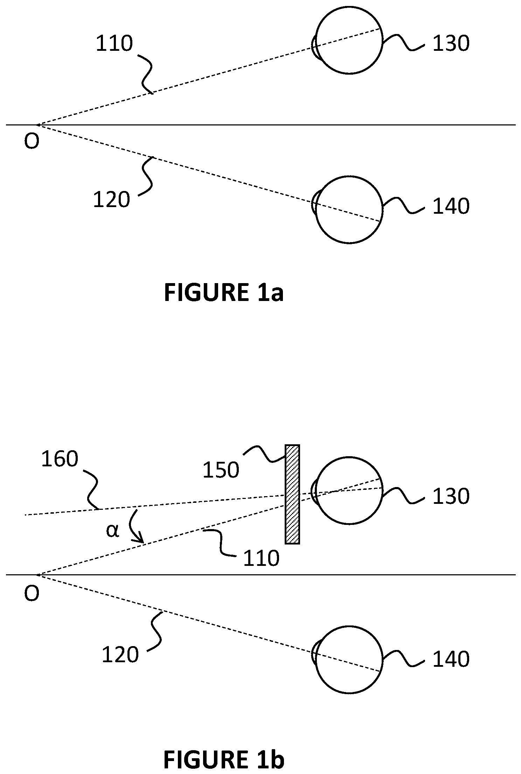

Concrètement, cela signifie qu'en situation de vision binoculaire normale, les deux yeux convergent vers le même point (en l'absence de strabisme), mais si on masque un œil, celui-ci dévie vers sa position de repos. Lorsqu'on démasque l'œil, il se recentre grâce au réflexe de fusion. Ce phénomène est illustré aux figures 1a et 1b. Sur la figure 1a, le sujet fixe une cible O en vision binoculaire et les axes visuels 110, 120 des deux yeux 130, 140 s'orientent vers cette cible O. Puis, comme représenté sur la figure 1b, l'un 130 des yeux du sujet (sur l'exemple de la figure 1b, l'œil droit) est caché grâce à un objet 150, par exemple un écran occultant. La fusion est alors empêchée, et l'œil masqué 130 prend une position de repos. Si le sujet présente une hétérophorie, l'axe visuel 160 de l'œil masqué 130 est décalé par rapport à l'axe visuel 110 du même œil 130 lorsqu'il est en position de fusion des images. Cette déviation peut être dans le plan horizontal et/ou dans le plan vertical. Sur l'exemple de la figure 1b, la déviation dans le plan horizontal est représentée par un angle α formé entre l'axe visuel 160 de l'œil masqué 130 en position de repos et l'axe visuel 110 du même œil 130 en vision binoculaire. Cet angle α représente le mouvement que l'œil doit effectuer pour repasser en vision binoculaire lors de la fusion. Lorsqu'il n'y a pas de décalage entre les axes visuels 110, 160 dans la position de repos et en vision binoculaire, on parle d'orthophorie.

L'hétérophorie ne peut donc être mise en évidence que lorsque la vision des deux yeux est dissociée, en l'absence de stimulus fusionnel (c'est-à-dire lorsque le réflexe fusionnel est empêché). Lorsqu'il y a absence de fusion en situation de vision binoculaire (les deux ouverts), il y a donc déviation manifeste d'un œil en position de convergence ou divergence par rapport au point fixé, on parle alors de tropie ou strabisme.

L'hétérophorie peut être verticale et/ou horizontale. Dans le cas d'une hétérophorie horizontale, lorsque la déviation de l'axe visuel se fait vers l'intérieur (vers le nez), on parle d'ésophorie, et lorsque la déviation de l'axe visuel se fait vers l'extérieur (vers la tempe), on parle d'exophorie. Dans le cas d'une hétérophorie verticale, lorsque la déviation de l'axe visuel se fait vers le haut, on parle d'hyperphorie, et lorsque la déviation de l'axe visuel se fait vers le bas, on parle d'hypophorie.

L'hétérophorie est très courante et concerne environ 75% de la population. Elle n'est généralement associée à aucune gêne particulière ou excessive (sauf si elle est verticale, ce qui n'est pas courant), car dans la grande majorité des cas, l'effort fusionnel (en horizontal) à accomplir pour faire concorder les axes des deux yeux en vision binoculaire est modéré et se fait sans difficulté. Toutefois, lorsque cet effort devient trop important, et que les réserves horizontales en convergence ou divergence du sujet sont insuffisantes, l'hétérophorie peut entraîner de la fatigue oculaire, des maux de tête, et même des problèmes de vision double, qui nécessitent une prise en charge par un orthoptiste pour permettre au sujet de retrouver un confort visuel à toutes les distances (entraînement visuel par exemple ou port de prismes notamment dans le cas des déviations verticales).

Afin d'apprécier la capacité de compensation d'une hétérophorie, les mesures de phories sont comparées aux valeurs des réserves fusionnelles. Les réserves fusionnelles permettent d'évaluer la capacité du sujet à faire converger ou diverger sa vision sans modification de l'accommodation (c'est-à-dire sans que la vision du sujet ne devienne floue) ou sans que sa vision ne devienne double. En d'autres termes, les réserves fusionnelles correspondent à la capacité musculaire du sujet à compenser la déviation des axes visuels lorsque les deux yeux sont au repos.

Les valeurs des réserves fusionnelles sont généralement déterminées en utilisant une tête de réfracteur dans laquelle sont introduits des prismes (un devant chaque œil). Les valeurs de déviation prismatique des prismes sont modifiées de manière à identifier le moment auquel le sujet ne peut plus accommoder (c'est-à-dire le moment où le sujet voit flou), puis le moment auquel le sujet n'est plus en mesure de converger ou de diverger (le sujet voit alors double) et enfin le moment où le sujet récupère une vision simple et nette (après avoir vu flou et/ou double - il est noté que certains sujets ont d'abord une vision floue puis double, mais d'autres sujets voient directement double). Les trois valeurs de déviations prismatique correspondant à la vision floue, à la vision double et à la vision simple et nette permettent de déterminer les valeurs des réserves fusionnelles.

Aujourd'hui, les valeurs de réserves fusionnelles sont déterminées manuellement, avec la mise en place d'un protocole rigoureux suivi par un praticien.

Il est par exemple connu d'utiliser une tête de réfracteur manuelle. Dans ce cas, le praticien fait varier les molettes de deux diasporamètres simultanément et note les valeurs de déviation prismatique associées à une vision floue du sujet (le cas échéant), puis à une vision double et enfin au retour à une vision simple et nette.

Il est également possible d'utiliser une tête de réfracteur automatique. Dans ce cas, les valeurs de déviation prismatiques des prismes sont modifiées par l'intermédiaire d'une seule molette qui permet d'obtenir des variations symétriques entre les deux yeux tout au long de l'examen. Ici aussi, le praticien agit sur la molette pour faire varier les valeurs de déviation prismatique des deux prismes.

Cependant, un tel protocole de détermination des valeurs des réserves fusionnelles est délicat et laborieux à mettre en œuvre. Pour que le test soit réalisé correctement, il faut le dérouler de façon fluide, sans interruption. Le praticien doit donc faire varier la ou les molettes des diasporamètres, retenir quand le sujet prévient des trois conditions, retenir les valeurs de déviation prismatique, avant de pouvoir noter ces valeurs sur le dossier patient à la fin du test. Une telle procédure est donc source d'erreurs et/ou de mauvaise réalisation, donc de manque de précision des valeurs.

En outre, avec une tête de réfracteur manuelle, le praticien doit additionner les valeurs des deux diasporamètres (en sachant qu'il n'est pas toujours évident d'augmenter de manière parfaitement symétrique les valeurs des deux diasporamètres, ce qui rend le calcul encore plus fastidieux) et ce tout en continuant d'augmenter les prismes à la même vitesse.

Ces étapes étant répétées pour effectuer les mesures horizontales, en vision de près VP et en vision de loin VL, le nombre de manipulations et le risque d'erreur dans l'interprétation correcte des réponses du sujet font de ce test une méthode longue et fastidieuse pour le praticien, ainsi que pour le sujet. Enfin, la variation des valeurs de déviation prismatique des prismes se fait à l'aide d'une ou deux molettes. La précision de la mesure peut donc être impactée par la variation des valeurs de déviation prismatique des prismes qui peut être moins régulière et par le temps qui peut être allongé. Le temps de réaction du praticien impacte également cette précision.

Tous les inconvénients et les contraintes mentionnés précédemment constituent un frein à un contrôle systématique de la vision binoculaire, souvent négligé à l'heure actuelle, lorsqu'un sujet vient passer des examens de la vue.

### RÉSUMÉ DE L'INVENTION

La présente invention vise donc à améliorer la détermination des valeurs des réserves fusionnelles de manière notamment à en améliorer la fiabilité mais aussi à en faciliter la détermination.

L'invention concerne alors un procédé de détermination d'au moins une valeur de réserves fusionnelles d'un sujet, le procédé étant mis en œuvre par un module de commande configuré pour commander une tête de réfracteur automatique, le procédé comprenant des étapes de :
- commande de la tête de réfracteur automatique pour placer, dans un premier emplacement de la tête de réfracteur automatique, un premier prisme associé à une première valeur de déviation prismatique,
- commande de la tête de réfracteur automatique pour placer, dans un deuxième emplacement de la tête de réfracteur automatique, un deuxième prisme associé à une deuxième valeur de déviation prismatique,
- positionnement de la tête de réfracteur automatique devant une tête du sujet de telle manière que le premier prisme soit positionné en face d'un premier œil du sujet et que le deuxième prisme soit positionné en face d'un deuxième œil du sujet,
- positionnement d'un optotype devant les yeux du sujet,
- commande de la tête de réfracteur automatique de sorte à faire défiler automatiquement, de manière croissante, la première valeur de déviation prismatique du premier prisme et la deuxième valeur de déviation prismatique du deuxième prisme, la première valeur de déviation prismatique du premier prisme étant égale à la deuxième valeur de déviation prismatique du deuxième prisme,
- réception d'une première indication associée à une vision double de l'optotype par le sujet,
- détermination d'une valeur de vision double correspondant à la première valeur de déviation prismatique du premier prisme et à la deuxième valeur de déviation prismatique du deuxième prisme à la réception de la première indication,
- suite à la détermination de la valeur de vision double, commande de la tête de réfracteur automatique de manière à faire défiler automatiquement, de manière décroissante, la première valeur de déviation prismatique du premier prisme et de la deuxième valeur de déviation prismatique du deuxième prisme, la première valeur de déviation prismatique du premier prisme étant égale à la deuxième valeur de déviation prismatique du deuxième prisme,
- réception d'une deuxième indication associée à une vision simple et nette de l'optotype par le sujet,
- détermination d'une valeur de recouvrement correspondant à la première valeur de déviation prismatique du premier prisme et à la deuxième valeur de déviation prismatique du deuxième prisme à la réception de la deuxième indication, et
- détermination d'au moins une valeur de réserves fusionnelles à partir de la valeur de vision double et de la valeur de recouvrement.

Par « défilement automatique », on entend un défilement (des valeurs de déviation prismatique) qui s'effectue à intervalles réguliers, sans l'intervention du praticien. Ce défilement automatique se produit donc de manière régulière (en d'autres termes, les valeurs de déviation prismatique varient de manière régulière).

De manière avantageuse selon l'invention, le défilement régulier et automatisé des valeurs de déviation prismatique du premier prisme et du deuxième prisme, simultanément, permet de déterminer de manière fiable les valeurs des réserves fusionnelles. En effet, comme le défilement est mis en œuvre de manière automatique, les valeurs de déviation prismatique varient de manière précise et régulière.

Par ailleurs, le praticien n'a pas besoin d'intervenir pour mettre en œuvre ce défilement. L'examen est donc plus facile à mettre en œuvre. De plus, comme le même processus de défilement est appliqué simultanément devant les yeux du sujet, cela permet de rendre l'examen plus agréable pour le sujet qui le subit. Le contrôle de la vision d'un sujet peut donc être mis en œuvre de manière simplifiée, rapide et efficace.

Outre les caractéristiques qui viennent d'être évoquées dans le paragraphe précédent, le procédé de détermination selon un aspect de l'invention peut présenter une ou plusieurs caractéristiques complémentaires parmi les suivantes, considérées individuellement ou selon toutes les combinaisons techniquement possibles :
- après le positionnement de l'optotype et préalablement à la commande de la tête de réfracteur automatique, il est prévu des étapes de :
   a) commande préalable de la tête de réfracteur automatique de sorte à faire défiler automatiquement, de manière croissante, la première valeur de déviation prismatique du premier prisme et de la deuxième valeur de déviation prismatique du deuxième prisme, la première valeur de déviation prismatique du premier prisme étant égale à la deuxième valeur de déviation prismatique du deuxième prisme,
   b) réception d'une indication préalable associée à une vision floue de l'optotype par le sujet, et
   c) détermination d'une valeur de vision floue correspondant à la première valeur de déviation prismatique du premier prisme et à la deuxième valeur de déviation prismatique du deuxième prisme à la réception de l'indication préalable,
   l'au moins une valeur de réserves fusionnelles étant déterminée à partir de la valeur de vision floue, de la valeur de vision double et de la valeur de recouvrement ;
- le défilement croissant est mis en œuvre de sorte que les premières valeurs de déviation prismatique du premier prisme sont espacées d'un pas fixe prédéfini et avec une fréquence prédéterminée comprise entre 0,5 Hz et 2 Hz et de sorte que les deuxièmes valeurs de déviation prismatique du deuxième prisme sont espacées du même pas fixe prédéfini et avec la même fréquence prédéterminée ;
- le défilement décroissant est mis en œuvre de sorte que les premières valeurs de déviation prismatique du premier prisme sont espacées du même pas fixe prédéfini et avec la même fréquence prédéterminée que lors du défilement croissant et de sorte que les deuxièmes valeurs de déviation prismatique du deuxième prisme sont espacées du même pas fixe prédéfini et avec la même fréquence prédéterminée que lors du défilement croissant ;
- après réception de la valeur de vision double, il est prévu une étape de défilement supplémentaire, de manière croissante et à partir de la valeur de vision double, de la première valeur de déviation prismatique du premier prisme et de la deuxième valeur de déviation prismatique du deuxième prisme, la première valeur de déviation prismatique du premier prisme et la deuxième valeur de déviation prismatique du deuxième prisme étant augmentées d'une valeur de déviation prismatique prédéterminée ;
- le défilement croissant et le défilement décroissant sont mis en œuvre avec une même vitesse de défilement en dioptrie prismatique par seconde pour la première valeur de déviation prismatique du premier prisme et la deuxième valeur de déviation prismatique du deuxième prisme ;
- la vitesse de défilement de la première valeur de déviation prismatique du premier prisme et de la deuxième valeur de déviation prismatique du deuxième prisme est d'une dioptrie prismatique par seconde ;
- la valeur de vision floue correspond à une somme de la première valeur de déviation prismatique du premier prisme et de la deuxième valeur de déviation prismatique du deuxième prisme lorsque, suite au défilement croissant, le sujet présente une vision floue de l'optotype ;
- la valeur de vision double correspond à une somme de la première valeur de déviation prismatique du premier prisme et de la deuxième valeur de déviation prismatique du deuxième prisme lorsque, suite au défilement croissant, le sujet présente une vision double de l'optotype ;
- la valeur de recouvrement correspond à une somme de la première valeur de déviation prismatique du premier prisme et de la deuxième valeur de déviation prismatique du deuxième prisme lorsque, suite au défilement décroissant, le sujet présente une vision simple et nette de l'optotype ;
- le premier prisme et le deuxième sont deux prismes horizontaux d'un même type parmi un prisme base interne et un prisme base externe ; et
- il est également prévu une étape d'affichage de l'au moins une valeur de réserves fusionnelles sur un moyen d'affichage.

Un autre aspect de l'invention concerne un dispositif de détermination d'au moins une valeur de réserves fusionnelles d'un sujet, le dispositif étant configuré pour commander une tête de réfracteur automatique et pour :
- commander la tête de réfracteur automatique pour placer, dans un premier emplacement de la tête de réfracteur automatique, un premier prisme associé à une première valeur de déviation prismatique,
- commander la tête de réfracteur automatique pour placer, dans un deuxième emplacement de la tête de réfracteur automatique, un deuxième prisme associé à une deuxième valeur de déviation prismatique,
- positionner la tête de réfracteur automatique devant une tête du sujet de telle manière que le premier prisme soit positionné en face d'un premier œil du sujet et que le deuxième prisme soit positionné en face d'un deuxième œil du sujet,
- positionner un optotype devant les yeux du sujet,
- commander la tête de réfracteur automatique de sorte à faire défiler automatiquement, de manière croissante, la première valeur de déviation prismatique du premier prisme et la deuxième valeur de déviation prismatique du deuxième prisme, la première valeur de déviation prismatique du premier prisme étant égale à la deuxième valeur de déviation prismatique du deuxième prisme,
- réceptionner une première indication associée à une vision double de l'optotype par le sujet,
- déterminer une valeur de vision double correspondant à la première valeur de déviation prismatique du premier prisme et à la deuxième valeur de déviation prismatique à la réception de la première indication,
- suite à la détermination de la valeur de vision double, commander la tête de réfracteur automatique de manière à faire défiler automatiquement, de manière décroissante, la première valeur de déviation prismatique du premier prisme et de la deuxième valeur de déviation prismatique du deuxième prisme, la première valeur de déviation prismatique du premier prisme étant égale à la deuxième valeur de déviation prismatique du deuxième prisme,
- réceptionner une deuxième indication associée à une vision simple et nette de l'optotype par le sujet,
- déterminer une valeur de recouvrement correspondant à la première valeur de déviation prismatique du premier prisme et à la deuxième valeur de déviation prismatique à la réception de la troisième indication, et
- déterminer au moins une valeur de réserves fusionnelles à partir de la valeur de vision double et de la valeur de recouvrement.

Un autre aspect de l'invention concerne un système de détermination d'au moins une valeur de réserves fusionnelles d'un sujet comprenant un dispositif de détermination tel que défini ci-dessus, une tête de réfracteur automatique et un écran de sélection.

Un programme informatique, mettant en œuvre tout ou partie du procédé décrit précédemment, installé sur un équipement préexistant, est en lui-même avantageux.

Ainsi, la présente invention vise également un programme informatique comportant des instructions pour la mise en œuvre de certaines étapes du procédé précédemment décrit, lorsque ce programme est exécuté par un processeur.

Ce programme peut utiliser n'importe quel langage de programmation (par exemple, un langage-objet ou autre), et être sous la forme d'un code source interprétable, d'un code partiellement compilé ou d'un code totalement compilé.

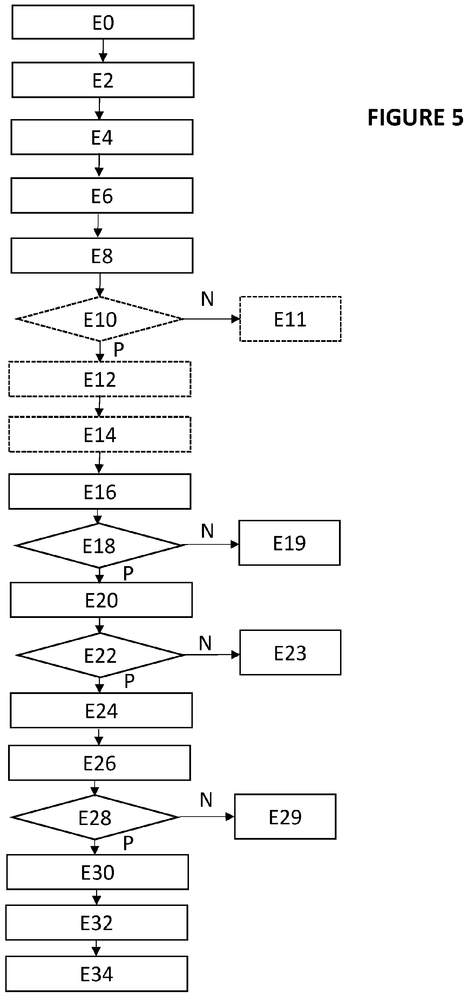

La figure 5 décrite en détail ci-après peut former l'organigramme de l'algorithme général d'un tel programme informatique.

L'invention et ses différentes applications seront mieux comprises à la lecture de la description qui suit et à l'examen des figures qui l'accompagnent.

### BRÈVE DESCRIPTION DES FIGURES

D'autres caractéristiques et avantages de l'invention ressortiront clairement de la description qui en est donnée ci-dessous, à titre indicatif et nullement limitatif, en référence aux figures annexées, parmi lesquelles :
[Fig. 1a], [Fig. 1b] Les figures 1a et 1b représentent un exemple d'hétérophorie chez un sujet ;
[Fig. 2] La figure 2 représente un système dans lequel un procédé de détermination d'au moins une valeur de réserves fusionnelles conforme à l'invention peut être mis en œuvre ;
[Fig. 3] La figure 3 représente un dispositif de détermination d'au moins une valeur de réserves fusionnelles conforme à l'invention ;
[Fig. 4a], [Fig. 4b] Les figures 4a et 4b représentent des exemples de prismes de l'état de la technique ;
[Fig. 5] La figure 5 représente, sous forme de logigramme, un exemple d'un procédé de détermination d'au moins une valeur de réserves fusionnelles selon l'invention ; et
[Fig. 6a], [Fig. 6b], [Fig. 6c], [Fig. 6d] Les figures 6a, 6b, 6c et 6d représentent des exemples d'informations et de propositions affichées sur l'écran de sélection pendant un procédé de détermination d'au moins une valeur de réserves fusionnelles selon l'invention.

Pour plus de clarté, les éléments identiques ou similaires sont repérés par des signes de référence identiques sur l'ensemble des figures.

### DESCRIPTION DÉTAILLÉE D'AU MOINS UN MODE DE RÉALISATION

La présente invention propose d'améliorer la détermination des valeurs des réserves fusionnelles d'un sujet afin de les rendre plus fiables mais aussi plus faciles à obtenir.

Pour cela, l'invention propose d'automatiser cette détermination des valeurs des réserves fusionnelles, en utilisant une tête de réfracteur automatique contrôlée par un dispositif de commande.

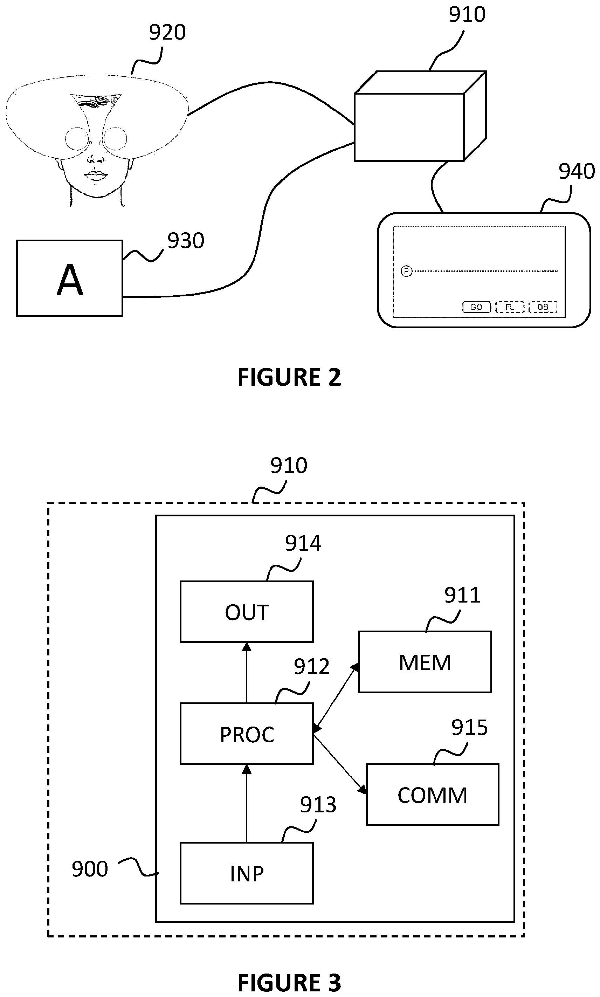

La figure 2 représente un exemple de système dans lequel un procédé de détermination d'au moins une valeur de réserves fusionnelles tel que décrit ci-après peut être mis en œuvre.

Le système de la figure 2 comprend un dispositif de commande (ou dispositif de contrôle) 910 comprenant un module de commande 900 (visible sur la figure 3) configuré pour commander une tête de réfracteur automatique 920. Dans l'exemple de la figure 2, le dispositif de commande 910 est relié à un écran de sélection d'un équipement utilisateur 940. Cet équipement utilisateur 940 se présente par exemple ici sous la forme d'une tablette. L'écran de sélection peut être un écran tactile, par le biais duquel le praticien peut fournir les données d'entrée au dispositif de commande 910. Les instructions relatives à la détermination d'au moins une valeur de réserves fusionnelles peut être affichées sur l'écran tactile. Bien entendu, l'écran tactile peut être remplacé par d'autres moyens de sélection et de réception d'instructions, y compris vocaux ou par des périphériques d'entrée tels qu'un joystick ou un pavé tactile.

Le dispositif de commande 910 est configuré pour commander un affichage sur cet écran de sélection et recevoir des données de sélection depuis l'écran de sélection. Dans cet exemple, le dispositif de commande 910 a la forme d'une « box » de connexion permettant de faire le lien entre différents équipements dédiés aux examens visuels. Bien entendu, d'autres modes de réalisation sont envisageables. En particulier, le dispositif de commande 910 peut comprendre l'écran de sélection. Dans certains modes de réalisation, l'écran de sélection peut être remplacé par tout autre système de sélection et de réception d'instructions (par exemple un écran et une souris, ou un écran et un système de reconnaissance vocale).

Le système de la figure 2 comprend en outre une tête de réfracteur automatique 920 configurée pour placer des verres optiques et/ou des prismes devant les yeux d'un sujet. La tête de réfracteur automatique 920 comprend deux emplacements correspondant respectivement aux deux yeux du sujet. Le dispositif de commande 910 est connecté à la tête de réfracteur automatique 920 et configuré pour contrôler le placement de verres ou de prismes dans chaque emplacement de la tête de réfracteur automatique 920.

Le système peut également comprendre un moyen d'affichage 930 (par exemple un écran). Le dispositif de commande 910 peut être connecté au moyen d'affichage 930 et configuré pour commander un affichage de données sur le moyen de sélection 930. Par exemple, le dispositif de commande 910 peut commander l'affichage d'un optotype sur le moyen d'affichage 930.

La connexion entre le dispositif de commande 910 et la tête de réfracteur automatique 920 et/ou entre le dispositif de commande 910 et le moyen d'affichage 930 et/ou entre le dispositif de commande 910 et l'équipement utilisateur 916 peut être filaire ou sans fil.

La figure 3 représente un exemple de dispositif de commande 910 permettant de mettre en œuvre au moins certaines étapes du procédé de détermination d'au moins une valeur de réserves fusionnelles conforme à l'invention.

Ici, le dispositif de commande 910 comporte un module de commande 900, comprenant une mémoire 911 pour stocker des instructions permettant la mise en œuvre du procédé et des données temporaires pour réaliser différentes étapes du procédé décrit dans la suite.

Le dispositif de commande 910 comprend également différents modules fonctionnels (par exemple un module de défilement, non représenté sur les figures) réalisés par exemple au moyen d'instructions de programme d'ordinateur mémorisées dans la mémoire 911 et conçues pour mettre en œuvre le module concerné lorsque ces instructions sont exécutées par le module de commande 900.

Le module de commande 900 comporte en outre un circuit 912. Ce circuit 912 peut être, par exemple, un processeur apte à interpréter des instructions sous la forme de programme informatique, une carte électronique dont les étapes du procédé de l'invention sont décrites dans le silicium, ou encore une puce électronique programmable comme une puce FPGA (pour « Field-Programmable Gate Array » en anglais).

Le module de commande 900 comporte également une interface d'entrée 913 pour la réception de données d'entrée, et une interface de sortie 914 pour la fourniture d'une ou plusieurs valeurs de réserves fusionnelles. Les données d'entrée peuvent être, par exemple, des données de démarrage de la mesure, des instructions correspondant à des sélections de proposition (par exemple une sélection d'une proposition de validation ou une sélection d'une proposition de valeur).

Le module de commande 900 peut aussi comprendre un module de communication 915 configuré pour communiquer avec un ou plusieurs équipements, notamment la tête de réfracteur automatique 920.

Le module de commande 900 est notamment configuré pour envoyer des signaux de commande à la tête de réfracteur automatique 920. Le dispositif de commande 910, comprenant ou étant relié à un écran de sélection tel que décrit précédemment, est configuré pour afficher des propositions à un utilisateur et à recevoir au moins une sélection d'au moins une des propositions affichées. A la réception d'un signal de commande, la tête de réfracteur automatique 920 est configurée pour placer au moins un prisme devant un œil du sujet. Comme indiqué précédemment, la tête de réfracteur automatique 920 comprend deux emplacements, chaque emplacement correspondant à un œil respectif, aptes à recevoir des prismes.

Pour la détermination d'au moins une valeur des réserves fusionnelles horizontales, les prismes utilisés sont des prismes de type prismes horizontaux.

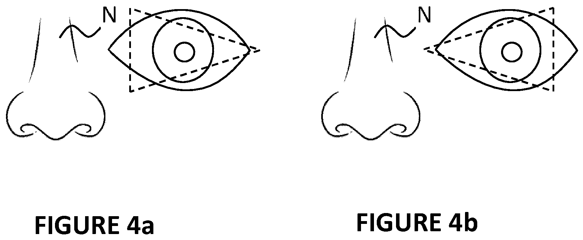

Les figures 4a et 4b représentent respectivement un prisme base interne et un prisme base externe, qui sont deux types de prismes horizontaux. Il est rappelé qu'un prisme est un verre dont les deux surfaces (ou, plus largement, les plans tangents aux deux surfaces du verre) sont inclinées l'une par rapport à l'autre (i.e. forment un angle fini non nul). La partie la plus mince au bord du prisme est appelée « sommet », et la partie la plus épaisse est appelée « base ».

Par « prisme horizontal », il est ainsi entendu un prisme dont l'axe sommet-base est horizontal (i.e. perpendiculaire à l'axe longitudinal du corps du patient, globalement dans l'axe passant par les centres des deux yeux du patient). Sur les figures 4a et 4b, l'œil représenté est l'œil gauche du patient. Le nez N du patient est également représenté sur les figures 4a et 4b. Un prisme base interne, tel que représenté à la figure 4a, a la base dirigée vers le nez du sujet. Un prisme base externe, tel que représenté à la figure 4b, a la base dirigée de l'autre côté par rapport au nez (vers la tempe du côté considéré).

Ainsi, tel que cela sera décrit ans la suite, un signal de commande (envoyé par le module de commande 900) peut par exemple comprendre :
- une première donnée indiquant devant quel œil un premier prisme doit être placé (c'est-à-dire dans quel emplacement de la tête de réfracteur automatique 920 doit être placé le premier prisme) ;
- une deuxième donnée indiquant devant quel œil (en pratique ici l'autre œil que celui concerné par le premier prisme) un deuxième prisme doit être placé (c'est-à-dire dans quel emplacement de la tête de réfracteur automatique 920 doit être placé le deuxième prisme) ; et
- une troisième donnée correspondant au type (base interne, base externe, base inférieure ou base supérieure) du premier prisme et du deuxième prisme à placer.

A la réception d'un tel signal de commande, la tête de réfracteur automatique 920 place, dans l'emplacement associé à la première donnée, un premier prisme du type indiqué par la troisième donnée et, dans l'emplacement associé à la deuxième donnée, un deuxième prisme du type indiqué par la troisième donnée.

L'exemple ci-dessus correspond à un signal de commande pour commander le placement de deux prismes, chacun devant un œil du sujet. En variante, le signal de commande peut commander des placements successifs d'une pluralité de prismes devant chaque œil du sujet.

Par exemple, le signal de commande peut comprendre un champ indiquant s'il concerne le placement d'un unique prisme ou de prismes successifs devant chaque œil du sujet. S'il concerne le placement de prismes successifs devant chaque œil du sujet, des champs supplémentaires comprenant une quatrième donnée correspondant au pas (i.e. l'écart, en dioptries prismatiques, entre deux valeurs successives de déviation prismatique des prismes) et une cinquième donnée correspondant à la fréquence de changement (i.e. la durée entre deux placements successifs de prismes). Ces champs peuvent être mis par défaut à des valeurs prédéfinies, par exemple 1 Δ et 1 seconde, qui sont modifiées uniquement si le signal de commande comprend d'autres valeurs.

Pour commander un défilement du premier prisme et du deuxième prisme ayant des valeurs de déviation prismatique successives (avec ici les mêmes valeurs pour le premier prisme et le deuxième prisme), le module de défilement peut : soit (comme dans le deuxième cas de figure ci-dessus) envoyer un seul signal de commande pour commander le placement successif des prismes (c'est-à-dire le placement successif de premiers prismes devant un œil du sujet et le placement successif de deuxièmes prismes devant l'autre œil du sujet), soit envoyer successivement plusieurs signaux de commande, chaque signal de commande commandant le placement d'un seul premier prisme et d'un seul deuxième prisme (comme dans le premier cas de figure ci-dessus). Dans ce dernier cas, les signaux de commande peuvent être envoyés par le module de commande 900 à la tête de réfracteur automatique 920 avec une fréquence et un pas prédéfinis.

A partir de ce dispositif de commande 910 muni du module de commande 900, il est possible de mettre en œuvre, de manière automatique, un procédé de détermination d'au moins une valeur de réserves fusionnelles horizontales.

La figure 5 représente, sous forme de logigramme, un exemple d'un tel procédé. Cet exemple du procédé de détermination est détaillé ci-dessous. Le procédé de détermination représenté sur la figure 5 est mis en œuvre par le module de commande 900. Contrairement aux méthodes connues de l'état de la technique et impliquant une molette que manœuvre un praticien, le procédé selon l'invention est mis en œuvre de manière totalement automatique.

Tout d'abord, à l'étape E0, le praticien met en route le système de la figure 2. Le moyen d'affichage 930 démarre également. Ce moyen d'affichage 930 affiche alors un écran général présentant l'examen de détermination d'au moins une valeur de réserves fusionnelles. Cet affichage présente donc le but de l'examen qui va être mis en œuvre ainsi que les consignes de cet examen que le sujet va devoir suivre pour la bonne mise en œuvre de la détermination des réserves fusionnelles.

Par exemple, ces consignes peuvent indiquer au sujet que, pour la détermination des réserves fusionnelles, différents prismes vont défiler devant ses yeux de manière continue et qu'il devra indiquer, dans un premier temps, quand l'optotype affiché apparaît flou (étape optionnelle comme cela est décrit dans la suite), puis, dans un deuxième temps, quand cet optotype apparaît double et enfin, dans un troisième temps, quand l'optotype redevient simple et net pour le sujet.

Après présentation du principe de la détermination d'au moins une valeur des réserves fusionnelles, le procédé de détermination se poursuit à l'étape E2. Lors de cette étape, le praticien sélectionne une proposition (par exemple, « commencer ») affichée sur l'écran de sélection pour commencer un examen de détermination d'au moins une valeur de réserves fusionnelles.

A la réception de la sélection de la proposition, l'écran de sélection affiche par exemple des champs, que le praticien peut remplir, correspondant, par exemple, à des valeurs de correction de la vision du sujet. En effet, la détermination des valeurs des réserves fusionnelles est mise en œuvre sur le sujet avec sa compensation optimale (i.e. avec verres de correction). Cela a pour but de limiter l'impact de l'accommodation sur la convergence des axes visuels et de permettre une bonne vision de la cible. En effet, lorsque que l'individu accommode afin de voir net, les axes visuels convergent. Ainsi, si un individu est mal ou non compensé en fonction des cas, il peut effectuer par exemple un effort accommodatif supplémentaire pour voir net, la détermination des valeurs des réserves fusionnelles s'en trouve impactée et ne reflète donc pas la réelle capacité de compensation du sujet. De plus, si le sujet est mal corrigé et donc doit analyser une image floue avant même le début du test, son désir de fusion est altéré, et les résultats obtenus aux différentes étapes de détermination des réserves fusionnelles peuvent être fortement impactées.

Une fois que le praticien a entré et validé ces valeurs, le module de commande 900 (relié à l'écran de sélection) envoie un signal de commande à la tête de réfracteur automatique 920 pour placer, devant les deux yeux du sujet, des verres correspondant aux valeurs de correction de la vision reçues. Alternativement, ces valeurs peuvent être directement récupérées, par exemple depuis un dossier numérique du sujet, ou d'un examen de la vue réalisé en amont sur un système piloté par le dispositif de commande 910.

Bien entendu, si la vision du sujet ne nécessite pas de correction, le procédé de détermination ne met pas en œuvre l'étape E2 et passe directement à l'étape suivante (étape E4).

Puis, à l'étape E4, l'écran de sélection affiche une ou plusieurs propositions correspondant à la mesure à effectuer (par exemple, le type de vision : vision de loin VL ou vision de près VP, un bouton de démarrage pour démarrer la mesure). Alternativement, une seule proposition pour commencer l'examen est affichée (les types de réserves fusionnelles et les types de vision étant alors selon un ordre prédéfini). A la réception des réponses du praticien, le module de commande 900 peut commander un écran pour afficher un optotype (sur le moyen d'affichage 930).

Dans le cas de la vision de loin VL, il s'agit généralement d'un optotype unique tandis que pour la vision de près VP, une suite d'optotypes sera affichée. Plus particulièrement, il s'agit par exemple d'une seule lettre isolée (ou un seul symbole isolé) en vision de loin VL ou d'un ensemble de lettres (ou de symboles), comme une ligne ou une colonne de lettres, en vision de près VP. De préférence, le symbole à lire doit être représenté de manière suffisamment fine (par exemple, au maximum 80% de l'acuité visuelle déterminée pendant un examen de vue du sujet afin d'obtenir une cible suffisamment précise) pour permettre de s'assurer que le patient donnera des réponses précises. En pratique, l'optotype est situé de l'autre côté de la compensation par rapport aux yeux du sujet.

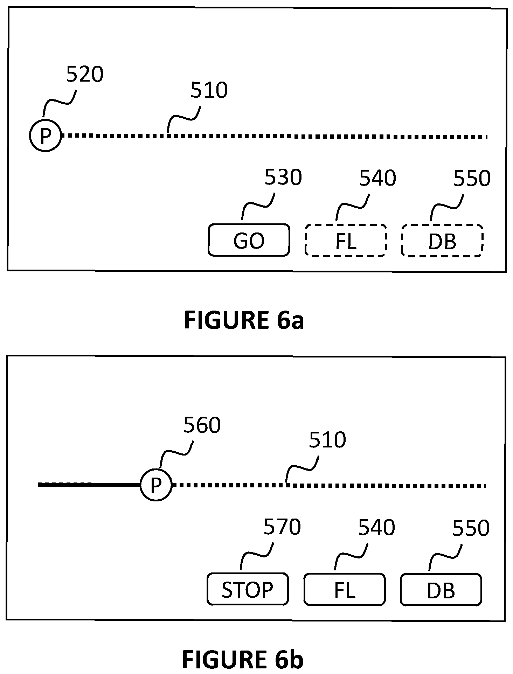

A l'issue de l'étape E4, le test à proprement parler permettant de déterminer au moins une valeur de réserves fusionnelles commence. La figure 6a illustre des exemples d'informations affichées sur l'écran de sélection lors de l'étape E4.

Comme illustré sur la figure 6a, avant de commencer à faire défiler les valeurs de déviation prismatique du premier prisme et du deuxième prisme, l'écran de sélection affiche une ligne d'avancement 510 comprenant une pluralité de positions représentant des valeurs possibles de déviation prismatiques (pour le premier prisme et le deuxième prisme). Il est important de noter que lors du défilement, le premier prisme et le deuxième prisme présentent la même valeur de déviation prismatique (simultanément). C'est pour cela qu'une seule valeur d'avancement 510 apparaît sur l'écran de sélection.

Par exemple ici, la ligne d'avancement 510 peut être une règle graduée dont chaque graduation correspond à une valeur de déviation prismatique (du premier prisme et du deuxième prisme). En pratique, la valeur de déviation prismatique affichée correspond par exemple à la somme de la valeur de déviation prismatique du premier prisme et de la valeur de déviation prismatique du deuxième prisme. Un symbole 520 est aussi affiché. Ce symbole peut par exemple correspondre à la valeur de déviation prismatique (du premier prisme et du deuxième prisme) positionnée devant les yeux du sujet.

Comme le montre la figure 6a, l'écran de sélection affiche ici trois propositions 530, 540 et 550.

La proposition 530 correspond à une instruction de démarrage du défilement des valeurs de déviation prismatique du premier prisme et du deuxième prisme. Dès lors que le praticien sélectionne cette proposition 530, le module de commande 900 envoie un ou plusieurs signaux de commande pour commander à la tête de réfracteur automatique 920 de faire défiler des prismes successifs (pour le premier prisme et le deuxième prisme).

La proposition 540 correspond à une instruction de vision floue de l'optotype par le sujet. La proposition 550 correspond à une instruction de vision double de l'optotype par le sujet. Tant que le défilement des prismes (i.e. du premier prisme et du deuxième prisme) n'a pas démarré, les propositions 540 et 550 ne sont pas sélectionnables.

Bien entendu, d'autres éléments peuvent être affichés sur l'écran de sélection. Par exemple, avant que la proposition 530 correspondant à l'instruction de démarrage du défilement des prismes, une consigne d'examen (comme lors de l'étape E0) peut à nouveau être affichée sur l'écran de sélection et/ou lue au sujet (par le praticien, ou par une voix de synthèse diffusée par un haut-parleur connectée au module de commande). Cette consigne peut être par exemple : « Le test va commencer. Prévenez-moi lorsque la lettre (la ligne) devient floue puis lorsqu'elle devient double. Ensuite, vous m'indiquerez le moment où elle redeviendra simple et nette. ».

Une proposition (non représentée) permettant l'arrêt à tout moment de l'examen peut également être affichée sur l'écran de sélection (afin de permettre l'arrêt du test si celui-ci n'est par exemple pas supporté par le patient).

Lorsque l'utilisateur sélectionne la proposition 530 correspondant à l'instruction de démarrage du défilement des prismes, le défilement des prismes (c'est-à-dire le défilement simultané des valeurs de déviation prismatique du premier prisme et du deuxième prisme) successifs commence.

Lors de l'étape E6, le module de commande 900 commande la tête de réfracteur automatique 920 pour placer le premier prisme dans un premier emplacement correspondant à un premier œil du sujet (par exemple l'œil droit). Le module de commande 900 commande également la tête de réfracteur automatique 920 pour placer le deuxième prisme dans un deuxième emplacement correspondant à un deuxième œil du sujet (par exemple ici l'œil gauche). En d'autres termes, un prisme est placé devant chaque œil du sujet (par l'intermédiaire de la tête de réfracteur automatique 920).

Il est à noter ici que le premier prisme et le deuxième prisme sont du même type. Ainsi, comme le procédé vise à déterminer une valeur des réserves fusionnelles horizontales, le premier prisme et le deuxième prisme sont des prismes de type horizontal (i.e. des prismes base interne ou base externe).

Par ailleurs, comme cela a été précisé précédemment, le premier prisme et le deuxième prisme présentent, tout au long de l'examen, la même valeur de déviation prismatique. En particulier, lors de cette étape E6, le premier prisme et le deuxième prisme présentent chacun une même valeur initiale de déviation prismatique. Cette valeur initiale de déviation prismatique est de préférence nulle.

Puis, lors d'étapes E8 successives, le module de commande 900 commande la tête de réfracteur automatique 920 pour faire défiler, de manière automatique, les valeurs de déviation prismatique du premier prisme et du deuxième prisme. Lors de ces étapes E8 successives, il s'agit d'un défilement croissant, c'est-à-dire que les valeurs de déviation prismatique du premier prisme et du deuxième prisme augmentent. Il est à noter que, lors de ce défilement croissant, la valeur de déviation prismatique du premier prisme est égale à la valeur de déviation prismatique du deuxième prisme. En d'autres termes, lors des étapes E8 successives, le premier prisme et le deuxième prisme présentent la même valeur de déviation prismatique à chaque instant de ce défilement croissant. Autrement dit encore, les valeurs de déviation prismatique du premier prisme et du deuxième prisme varient simultanément lors du défilement croissant.

Ce défilement croissant est mis en œuvre avec une vitesse de défilement constante. Cette vitesse de défilement est par exemple de l'ordre de 1 dioptrie par œil par seconde.

Le premier prisme et le deuxième prisme ont alors des valeurs successives croissantes de déviation prismatique. Ces valeurs successives sont espacées d'un pas prédéfini, par exemple 0,5 Δ ou 1 Δ. Cela permet alors de tester des valeurs successives de déviation prismatique suffisamment proches pour obtenir une valeur de vision flou, de vision double et de recouvrement (tel que décrit ci-après) suffisamment précises. Il est noté qu'il est compliqué, voire impossible, de faire défiler des prismes de manière régulière dans les méthodes manuelles. Cela engendre une durée allongée de la mesure, qui fausse la mesure. Ces problèmes sont évités avec le procédé automatique conforme à l'invention.

Ces valeurs successives de déviation prismatique sont également placées devant les yeux du sujet selon une fréquence prédéterminée. Cette fréquence prédéterminée est par exemple comprise entre 0,5 Hz et 2 Hz (ce qui signifie que la durée séparant deux valeurs de déviation prismatique successives est par exemple d'une seconde, de 1,5 seconde, de deux secondes, etc). Cela signifie alors que l'écart temporel entre deux placements consécutifs (pour le premier prisme et pour le deuxième prisme) est fixe et compris entre 0,5 seconde et 2 secondes. Par exemple, la fréquence peut être égale à 1 Hz. De telles valeurs permettent un défilement suffisamment rapide pour que la durée de détermination des valeurs de réserves fusionnelles reste raisonnable, mais suffisamment long pour qu'il n'y ait pas (trop) de retard entre le moment où le sujet détecte que l'optotype devient flou, double ou que l'optotype est à nouveau simple et net.

Ce défilement régulier des valeurs successives de déviation prismatique pour le premier prisme et le deuxième prisme permet de garantir un examen régulier et automatisé pour déterminer les valeurs des réserves fusionnelles. Ces dernières sont donc déterminées de manière plus fiable. De plus, comme le même processus de défilement est appliqué simultanément devant les yeux du sujet, cela permet de rendre l'examen plus agréable pour le sujet qui le subit.

Comme mentionné précédemment, le module de commande 900 peut commander le défilement des prismes (i.e. du premier prisme et du deuxième prisme) en envoyant un ou plusieurs signaux de commande à la tête de réfracteur automatique 920.

La figure 6b illustre l'écran de sélection lors des étapes E8 successives de défilement croissant des valeurs de déviation prismatique du premier prisme et du deuxième prisme. Un symbole 560 correspondant à la valeur courante de déviation prismatique du premier prisme et du deuxième prisme est affiché sur l'écran de sélection. Ici, il est par exemple superposé à la ligne d'avancement 510. Ainsi, des symboles successifs 560 peuvent défiler sur l'écran pour faire apparaître la valeur courante de déviation prismatique du premier prisme et du deuxième prisme, ce qui permet au praticien de savoir très rapidement et simplement où en est le défilement des prismes. Pendant le défilement des prismes, la proposition 530 (de démarrage du test) a été remplacée par une proposition 570 correspondant à une instruction d'arrêt du test. Cette proposition 570 est sélectionnable (si par exemple le sujet ne se sent pas bien et/ou demande l'arrêt de l'examen). Les propositions 540 et 550 (décrites précédemment) sont également sélectionnables.

Dans certains modes de réalisation, pendant le défilement des prismes (i.e. du premier prisme et du deuxième prisme), les valeurs de déviation prismatique des prismes sont dictées, par exemple via un haut-parleur relié au module de commande. Ainsi, dès que de nouvelles valeurs de déviation prismatique (pour le premier prisme et pour le deuxième prisme) sont placées dans la tête de réfracteur automatique, un son indiquant la valeur de déviation prismatique correspondante est émis.

Tant qu'aucune indication concernant une vision floue de l'optotype par le sujet n'est reçue, le défilement croissant des valeurs de déviation prismatique du premier prisme et du deuxième prisme se poursuit. Ce défilement croissant se poursuit jusqu'à ce que la valeur de déviation prismatique atteigne une valeur maximale. Cette valeur maximale est par exemple de l'ordre de 20 dioptries (pour le premier prisme d'une part, et pour le deuxième prisme d'autre part).

Si, à l'étape E10, aucune indication concernant une vision floue de l'optotype par le sujet n'est reçue, le test de détermination d'au moins une valeur de réserves fusionnelles est arrêtée (à l'étape E11). Il faudra alors recommencer le test dès l'étape E0 si au moins une valeur de réserves fusionnelles doit être déterminée.

Une indication concernant la vision floue de l'optotype est par exemple reçue via l'écran de sélection (par exemple lorsque le praticien sélectionne la proposition 540 sur l'écran de sélection) ou via n'importe quel autre moyen (par exemple via un système comprenant un micro et un module de détection de voix, lorsque le praticien ou le sujet dit « FLOU »).

A la réception d'une indication sur le fait que le sujet voit de manière floue l'optotype, la valeur courante de déviation prismatique (i.e. la valeur de déviation prismatique du premier prisme placé dans le premier emplacement et la valeur de déviation prismatique du deuxième prisme placé dans le deuxième emplacement lors de la réception de l'indication) est enregistrée comme valeur de vision floue (étape E12).

La valeur de vision floue correspond par exemple ici à la somme des valeurs de déviation prismatique du premier prisme et du deuxième prisme lors de la réception de l'indication concernant le fait que le sujet voit l'optotype de manière floue. En variante, la valeur de vision floue peut être directement la valeur de déviation prismatique du premier prisme et du deuxième prisme lors de la réception de l'indication concernant le fait que le sujet voit l'optotype de manière floue.

A l'étape E14, cette valeur de vision floue est mémorisée, par exemple dans la mémoire 911.

Puis, le défilement croissant se poursuit (étapes E16 successives similaires aux étapes E8 décrites précédemment).

Il est à noter que la détermination de la valeur de vision floue est optionnelle (c'est-à-dire que les étapes E10 à E14 peuvent être omises). En effet, dans certains cas, il est attendu que le patient ne soit pas en mesure de percevoir une version floue de l'optotype. C'est par exemple le cas pour la détermination des réserves fusionnelles en divergence en vision de loin VL (où le patient perçoit directement une version double de l'optotype, avec une correction permettant d'obtenir la meilleure acuité visuelle possible en vision de loin avec l'accommodation relâchée au maximum). Pour ces cas de figure, les étapes E8 et E16 sont fusionnées.

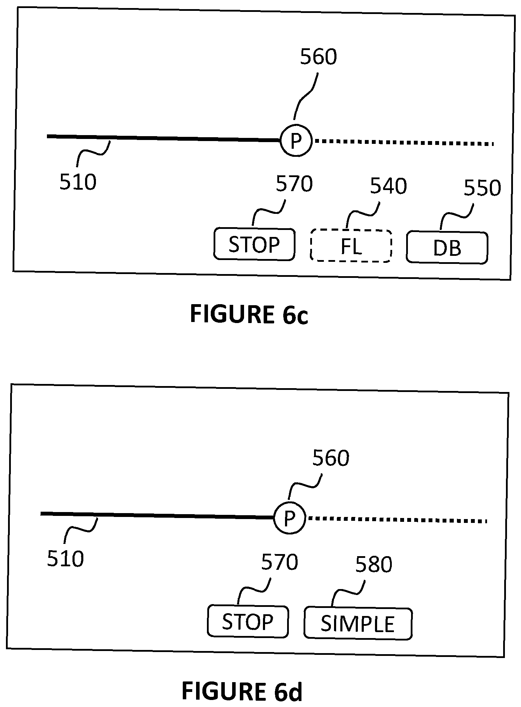

La figure 6c illustre l'écran de sélection lors des étapes E16 successives de défilement croissant des valeurs de déviation prismatique du premier prisme et du deuxième prisme. Le symbole 560 correspondant à la valeur courante de déviation prismatique du premier prisme et du deuxième prisme est toujours affiché sur l'écran de sélection. Ici aussi, il est par exemple superposé à la ligne d'avancement 510. Ici aussi, des symboles successifs 560 peuvent défiler sur l'écran pour faire apparaître la valeur courante de déviation prismatique du premier prisme et du deuxième prisme, ce qui permet au praticien de savoir très rapidement et simplement où en est le défilement des prismes. Pendant la poursuite de défilement des prismes, la proposition 570 correspondant à une instruction d'arrêt du test est sélectionnable (si par exemple le sujet ne se sent pas bien et/ou demande l'arrêt de l'examen - en effet le défilement de prismes devant les yeux du sujet en vue de l'obtention d'une vision double peut être désagréable et, dans certains cas, causer un trouble de type mal de tête ou nausées). Les propositions 550 est également sélectionnable. La proposition 540 n'est en revanche elle plus sélectionnable car elle a été sélectionnée à l'étape E10 (suite à l'indication reçue).

Tant qu'aucune indication concernant une vision double de l'optotype par le sujet n'est reçue, le défilement croissant des valeurs de déviation prismatique du premier prisme et du deuxième prisme se poursuit. Ce défilement croissant se poursuit jusqu'à ce que la valeur de déviation prismatique atteigne une valeur maximale. Cette valeur maximale est par exemple ici aussi de l'ordre de 20 dioptries (pour le premier prisme d'une part, et pour le deuxième prisme d'autre part).

Si à l'étape E18, aucune indication concernant une vision double de l'optotype par le sujet n'est reçue, le test de détermination d'au moins une valeur de réserves fusionnelles est arrêté (à l'étape E19). Il faudra alors recommencer le test dès l'étape E0 si au moins une valeur de réserves fusionnelles doit être déterminée.

En variante, l'arrêt du test peut également se produire lorsque le praticien sélectionne, sur l'écran de sélection, une proposition (non représentée sur les figures) indiquant que le sujet n'a pas vu l'optotype de manière double.

Une indication concernant la vision double de l'optotype est par exemple reçue via l'écran de sélection (par exemple lorsque le praticien sélectionne la proposition 550 sur l'écran de sélection) ou via n'importe quel autre moyen (par exemple via un système comprenant un micro et un module de détection de voix, lorsque le praticien ou le sujet dit « DOUBLE »).

A la réception d'une indication sur le fait que le sujet voit de manière double l'optotype, la valeur courante de déviation prismatique (i.e. la valeur de déviation prismatique du premier prisme placé dans le premier emplacement et la valeur de déviation prismatique du deuxième prisme placé dans le deuxième emplacement lors de la réception de l'indication) est enregistrée comme valeur de vision double (étape E20).

La valeur de vision double correspond par exemple ici à la somme des valeurs de déviation prismatique du premier prisme et du deuxième prisme lors de la réception de l'indication concernant le fait que le sujet voit l'optotype de manière double.

A l'étape E22, le module de commande 900 procède à une validation de cette valeur de vision double. Plus particulièrement, le module de commande 900 compare la valeur de vision double obtenue à la valeur maximale de déviation prismatique introduite précédemment (i.e. par exemple ici de l'ordre de 20 dioptries pour chacun du premier prisme et du deuxième prisme). Si la valeur de vision double est trop proche de cette valeur maximale, c'est-à-dire avec un écart inférieur à quelques dioptries, la valeur de vision double est validée (et mémorisée par exemple dans la mémoire 911), mais le test est arrêté (à l'étape E23). Par exemple, un écart inférieur à 5 dioptries est utilisé ici pour la validation de la valeur de vision double.

Si la valeur de vision double est validée, le procédé se poursuit par une étape E24 de défilement croissant supplémentaire. Lors de cette étape, la valeur de déviation prismatique du premier prisme et du deuxième prisme est incrémentée d'une valeur de déviation prismatique prédéterminée à partir de la valeur de vision double. Cette valeur de déviation prismatique est de l'ordre de quelques dioptries, par exemple de 4 à 6 dioptries.

Cette étape E24 est avantageuse car elle permet de confirmer que le sujet voit l'optotype de manière double et de s'assurer que la vision binoculaire est bien définitivement brisée. En effet, comme l'examen mis en œuvre peut être associé à un ressenti d'inconfort pour le sujet et peut potentiellement entraîner une fatigue oculaire, il n'est pas exclu que le sujet indique voir l'optotype de manière double mais que cela soit dû à une fatigue passagère. La vision binoculaire peut finalement ne pas être définitivement brisée et la valeur de vision double obtenue à l'étape E20 n'est pas satisfaisante. L'étape E24 de défilement croissant supplémentaire permet donc de s'assurer que le sujet voit effectivement l'optotype de manière double. Cela permet alors de renforcer la fiabilité des valeurs des réserves fusionnelles déterminées..

Suite à la détermination de la valeur de vision double, lors d'étapes E26 successives, le module de commande 900 commande la tête de réfracteur automatique 920 pour faire défiler, de manière automatique, les valeurs de déviation prismatique du premier prisme et du deuxième prisme. Lors de ces étapes E26 successives, il s'agit d'un défilement décroissant, c'est-à-dire que les valeurs de déviation prismatique du premier prisme et du deuxième prisme diminuent. Il est à noter que, lors de ce défilement décroissant, la valeur de déviation prismatique du premier prisme est égale à la valeur de déviation prismatique du deuxième prisme. En d'autres termes, lors des étapes E26 successives, le premier prisme et le deuxième prisme présentent la même valeur de déviation prismatique à chaque instant de ce défilement décroissant. Autrement dit encore, les valeurs de déviation prismatique du premier prisme et du deuxième prisme varient simultanément lors du défilement décroissant.

Ce défilement décroissant est mis en œuvre à partir de la valeur de vision double ou à partir de la valeur de vision double incrémentée de la valeur de déviation prismatique prédéterminée (tel que décrit précédemment à l'étape E24).

Ce défilement décroissant est mis en œuvre avec une vitesse de défilement constante. Cette vitesse de défilement est par exemple de l'ordre de 1 dioptrie par œil par seconde. De préférence, la vitesse de défilement décroissant est la même que celle mis en œuvre pour le défilement croissant décrit précédemment (étapes E8 et E16).

Le premier prisme et le deuxième prisme ont alors des valeurs successives décroissantes de déviation prismatique. Ces valeurs successives sont espacées d'un pas prédéfini, par exemple 0,5 Δ ou 1 Δ. Ces valeurs successives de déviation prismatique sont également placées devant les yeux du sujet selon une fréquence prédéterminée (par exemple chaque seconde, chaque 1,5 seconde, toutes les deux secondes, etc). De préférence, les caractéristiques utilisées pour le défilement décroissant (pas, fréquence, vitesse) sont les mêmes que celles mises en œuvre pour le défilement croissant décrit précédemment.

Ce défilement régulier des valeurs successives de déviation prismatique pour le premier prisme et le deuxième prisme permet, ici aussi, de garantir un examen régulier et automatisé pour déterminer les valeurs des réserves fusionnelles. Ces dernières sont donc déterminées de manière plus fiable. De plus, comme le même processus de défilement est appliqué simultanément devant les yeux du sujet, cela permet de rendre l'examen plus agréable pour le sujet qui le subit. Par ailleurs, dans le cadre de la présente invention, à la fin du processus de défilement, le retrait des prismes est effectué de manière rapide et précise, ce qui permet également de réduire la durée d'inconfort ressenti par le sujet.

Comme mentionné précédemment, le module de commande 900 peut commander le défilement (décroissant) des prismes (i.e. du premier prisme et du deuxième prisme) en envoyant un ou plusieurs signaux de commande à la tête de réfracteur automatique 920.

La figure 6d illustre l'écran de sélection lors des étapes E26 successives de défilement décroissant des valeurs de déviation prismatique du premier prisme et du deuxième prisme. Le symbole 560 correspondant à la valeur courante de déviation prismatique du premier prisme et du deuxième prisme est toujours affiché sur l'écran de sélection. Ici aussi, il est par exemple superposé à la ligne d'avancement 510. Ainsi, des symboles successifs 560 peuvent défiler sur l'écran pour faire apparaître la valeur courante de déviation prismatique du premier prisme et du deuxième prisme, ce qui permet au praticien de savoir très rapidement et simplement où en est le défilement des prismes. Pendant le défilement décroissant des prismes, la proposition 570 correspondant à une instruction d'arrêt du test est sélectionnable (si par exemple le sujet ne se sent pas bien et/ou demande l'arrêt de l'examen). Une proposition 580 est affichée sur l'écran de sélection. La proposition 580 correspond à une instruction de vision simple et nette de l'optotype par le sujet (i.e. une instruction selon laquelle le sujet voit à nouveau l'optotype de façon simple et nette). Cette proposition 580 est sélectionnable pendant le défilement décroissant

Dans certains modes de réalisation, pendant le défilement (décroissant) des prismes, les valeurs de déviation prismatique de prismes sont dictées, par exemple via un haut-parleur relié au module de commande. Ainsi, dès que de nouvelles valeurs de déviation prismatique (pour le premier prisme et pour le deuxième prisme) sont placées dans la tête de réfracteur automatique, un son indiquant la valeur de déviation prismatique correspondante est émis.

Tant qu'aucune indication concernant une vision simple et nette de l'optotype par le sujet n'est reçue, le défilement décroissant des valeurs de déviation prismatique du premier prisme et du deuxième prisme se poursuit. Ce défilement décroissant se poursuit jusqu'à ce que la valeur de déviation prismatique atteigne une valeur minimale. Cette valeur minimale est par exemple nulle (pour le premier prisme d'une part, et pour le deuxième prisme d'autre part).

Si à l'étape E28, aucune indication concernant une vision simple et nette de l'optotype par le sujet n'est reçue alors que la valeur de déviation prismatique atteint la valeur minimale, une indication indiquant que le sujet ne présente pas une vision simple et nette est reçue par le module de commande 900. Cette indication est par exemple reçue via l'écran de sélection (par exemple lorsque le praticien sélectionne une proposition correspondante (non représentée) sur l'écran de sélection). Le test de détermination d'au moins une valeur de réserves fusionnelles est alors arrêté (à l'étape E29). Il faut alors recommencer le test dès l'étape E0 si au moins une valeur de réserves fusionnelles doit être déterminée.

Une indication concernant la vision simple et nette de l'optotype est par exemple reçue via l'écran de sélection (par exemple lorsque le praticien sélectionne la proposition 580 sur l'écran de sélection) ou via n'importe quel autre moyen (par exemple via un système comprenant un micro et un module de détection de voix, lorsque le praticien ou le sujet dit « SIMPLE »).

A la réception d'une indication sur le fait que le sujet voit de manière simple et nette l'optotype, la valeur courante de déviation prismatique (i.e. la valeur de déviation prismatique du premier prisme placé dans le premier emplacement et la valeur de déviation prismatique du deuxième prisme placé dans le deuxième emplacement lors de la réception de l'indication) est enregistrée comme valeur de recouvrement (étape E30).

La valeur de recouvrement correspond par exemple ici à la somme des valeurs de déviation prismatique du premier prisme et du deuxième prisme lors de la réception de l'indication concernant le fait que le sujet voit à nouveau l'optotype de manière simple et nette.

Comme le montre le logigramme de la figure 5, le procédé de détermination se poursuit ensuite à l'étape E32. Lors de cette étape, le module de commande 900 détermine au moins une valeur de réserves fusionnelles à partir de la valeur de vision floue déterminée à l'étape E14, de la valeur de vision double déterminée à l'étape E22 et de la valeur de recouvrement déterminée à l'étape E30. Les valeurs de réserves fusionnelles sont par exemple directement la valeur de vision floue, la valeur de vision double et la valeur de recouvrement obtenues. En d'autres termes, la valeur de vision floue déterminée à l'étape E14, la valeur de vision double déterminée à l'étape E22 et la valeur de recouvrement déterminée à l'étape E30 représentent les valeurs des réserves fusionnelles recherchées.

En variante, dans les cas décrits précédemment où seule(s) la valeur de vision floue et/ou la valeur de vision double a(ont) été validée(s) (et que le test a été arrêté ensuite), celle(s)-ci représente(nt) une(des) valeur(s) de réserves fusionnelles.

Enfin, à l'étape E34, le module de commande 900 commande l'affichage, sur le moyen d'affichage 930, d'un récapitulatif des valeurs de réserves fusionnelles déterminées. En pratique, les valeurs de réserves fusionnelles déterminées sont par exemple représentées sur une ligne d'avancement 510 avec des symboles différents (pour permettre de les distinguer). Ce récapitulatif affiché peut également comprendre les valeurs des phories mesurées par l'intermédiaire d'un procédé approprié (non décrit ici).

De manière avantageuse selon l'invention, le procédé de détermination décrit s'applique de manière à déterminer les valeurs de réserves fusionnelles en divergence en vision de loin VL, en convergence en vision de loin VL, en divergence en vision de près VP et en convergence en vision de près VP. De préférence, la détermination est mise en œuvre en commençant par la détermination des réserves fusionnelles en base interne en divergence, puis en convergence. Cela permet alors de réduire l'accommodation avant de la solliciter. Par ailleurs, il est courant de commencer par la détermination des réserves fusionnelles en vision de loin VL puis de poursuivre par la détermination des réserves fusionnelles en vision de près VP. Toutefois, cet ordre de détermination (vision de loin VL, vision de près VP) est indifférent. Ainsi, lorsque toutes les déterminations sont effectuées, le module de commande 900 commande la mémorisation de douze valeurs de réserves fusionnelles pour le sujet concerné.

Il est à noter que l'examen de détermination des valeurs de réserves fusionnelles peut être interrompu à tout moment par le praticien ou le sujet si cela est nécessaire. Cela peut notamment intervenir si cela n'est pas une expérience agréable pour le sujet. Une indication d'arrêt est par exemple reçue via l'écran de sélection (par exemple lorsque le praticien sélectionne la proposition 570 sur l'écran de sélection) ou via n'importe quel autre moyen (par exemple via un système comprenant un micro et un module de détection de voix, lorsque le praticien ou le sujet dit « STOP »).

Finalement, de manière avantageuse selon l'invention, le défilement régulier et automatisé des valeurs de déviation prismatique du premier prisme et du deuxième prisme, simultanément, permet de déterminer de manière fiable les valeurs des réserves fusionnelles.

Par ailleurs, le praticien n'a pas besoin d'intervenir pour mettre en œuvre ce défilement. L'examen est donc plus facile à mettre en œuvre. De plus, comme le même processus de défilement est appliqué simultanément devant les yeux du sujet, cela permet de rendre l'examen plus agréable pour le sujet qui le subit. Le contrôle de la vision d'un sujet peut donc être mis en œuvre de manière simplifiée, rapide et efficace.

## Revendications

1. Procédé de détermination d'au moins une valeur de réserves fusionnelles d'un sujet, le procédé étant mis en œuvre par un module de commande (900) configuré pour commander une tête de réfracteur automatique (920), le procédé comprenant des étapes de :
- commande de la tête de réfracteur automatique (920) pour placer, dans un premier emplacement de la tête de réfracteur automatique (920), un premier prisme associé à une première valeur de déviation prismatique,
- commande de la tête de réfracteur automatique (920) pour placer, dans un deuxième emplacement de la tête de réfracteur automatique (920), un deuxième prisme associé à une deuxième valeur de déviation prismatique,
- positionnement de la tête de réfracteur automatique (920) devant une tête du sujet de telle manière que le premier prisme soit positionné en face d'un premier œil du sujet et que le deuxième prisme soit positionné en face d'un deuxième œil du sujet,
- positionnement d'un optotype devant les yeux du sujet,
- commande de la tête de réfracteur automatique (920) de sorte à faire défiler automatiquement, de manière croissante, la première valeur de déviation prismatique du premier prisme et la deuxième valeur de déviation prismatique du deuxième prisme, la première valeur de déviation prismatique du premier prisme étant égale à la deuxième valeur de déviation prismatique du deuxième prisme,
- réception d'une première indication associée à une vision double de l'optotype par le sujet,
- détermination d'une valeur de vision double correspondant à la première valeur de déviation prismatique du premier prisme et à la deuxième valeur de déviation prismatique du deuxième prisme à la réception de la première indication,
- commande de la tête de réfracteur automatique (920) de sorte à mettre en œuvre un défilement supplémentaire, de manière croissante et à partir de la valeur de vision double, de la première valeur de déviation prismatique du premier prisme et de la deuxième valeur de déviation prismatique du deuxième prisme, la première valeur de déviation prismatique du premier prisme et la deuxième valeur de déviation prismatique du deuxième prisme étant augmentées d'une valeur de déviation prismatique prédéterminée,
- suite à la détermination de la valeur de vision double, commande de la tête de réfracteur automatique (920) de manière à faire défiler automatiquement, de manière décroissante, la première valeur de déviation prismatique du premier prisme et de la deuxième valeur de déviation prismatique du deuxième prisme, la première valeur de déviation prismatique du premier prisme étant égale à la deuxième valeur de déviation prismatique du deuxième prisme,
- réception d'une deuxième indication associée à une vision simple et nette de l'optotype par le sujet,
- détermination d'une valeur de recouvrement correspondant à la première valeur de déviation prismatique du premier prisme et à la deuxième valeur de déviation prismatique du deuxième prisme à la réception de la deuxième indication, et
- détermination d'au moins une valeur de réserves fusionnelles à partir de la valeur de vision double et de la valeur de recouvrement.

2. Procédé selon la revendication 1, dans lequel, après le positionnement de l'optotype et préalablement à la commande de la tête de réfracteur automatique (920), il est prévu des étapes de :
- commande préalable de la tête de réfracteur automatique (920) de sorte à faire défiler automatiquement, de manière croissante, la première valeur de déviation prismatique du premier prisme et de la deuxième valeur de déviation prismatique du deuxième prisme, la première valeur de déviation prismatique du premier prisme étant égale à la deuxième valeur de déviation prismatique du deuxième prisme,
- réception d'une indication préalable associée à une vision floue de l'optotype par le sujet,
- détermination d'une valeur de vision floue correspondant à la première valeur de déviation prismatique du premier prisme et à la deuxième valeur de déviation prismatique du deuxième prisme à la réception de l'indication préalable,
l'au moins une valeur de réserves fusionnelles étant déterminée à partir de la valeur de vision floue, de la valeur de vision double et de la valeur de recouvrement.

3. Procédé selon la revendication 1 ou 2, dans lequel le défilement croissant est mis en œuvre de sorte que les premières valeurs de déviation prismatique du premier prisme sont espacées d'un pas fixe prédéfini et avec une fréquence prédéterminée comprise entre 0,5 Hz et 2 Hz et de sorte que les deuxièmes valeurs de déviation prismatique du deuxième prisme sont espacées du même pas fixe prédéfini et avec la même fréquence prédéterminée, et
dans lequel le défilement décroissant est mis en œuvre de sorte que les premières valeurs de déviation prismatique du premier prisme sont espacées du même pas fixe prédéfini et avec la même fréquence prédéterminée que lors du défilement croissant et de sorte que les deuxièmes valeurs de déviation prismatique du deuxième prisme sont espacées du même pas fixe prédéfini et avec la même fréquence prédéterminée que lors du défilement croissant.

4. Procédé selon l'une quelconque des revendications 1 à 3, dans lequel le défilement croissant et le défilement décroissant sont mis en œuvre avec une même vitesse de défilement en dioptrie prismatique par seconde pour la première valeur de déviation prismatique du premier prisme et la deuxième valeur de déviation prismatique du deuxième prisme.

5. Procédé selon la revendication 4, dans lequel la vitesse de défilement de la première valeur de déviation prismatique du premier prisme et de la deuxième valeur de déviation prismatique du deuxième prisme est d'une dioptrie prismatique par seconde.

6. Procédé selon l'une quelconque des revendications 2 à 5, dans lequel la valeur de vision floue correspond à une somme de la première valeur de déviation prismatique du premier prisme et de la deuxième valeur de déviation prismatique du deuxième prisme lorsque, suite au défilement croissant, le sujet présente une vision floue de l'optotype.

7. Procédé selon l'une quelconque des revendications 1 à 6, dans lequel la valeur de vision double correspond à une somme de la première valeur de déviation prismatique du premier prisme et de la deuxième valeur de déviation prismatique du deuxième prisme lorsque, suite au défilement croissant, le sujet présente une vision double de l'optotype.

8. Procédé selon l'une quelconque des revendications 1 à 7, dans lequel la valeur de recouvrement correspond à une somme de la première valeur de déviation prismatique du premier prisme et de la deuxième valeur de déviation prismatique du deuxième prisme lorsque, suite au défilement décroissant, le sujet présente une vision simple et nette de l'optotype.

9. Procédé selon l'une quelconque des revendication 1 à 8, dans lequel le premier prisme et le deuxième sont deux prismes horizontaux d'un même type parmi un prisme base interne et un prisme base externe.

10. Procédé selon l'une quelconque des revendications 1 à 9, dans lequel il est également prévu une étape d'affichage de l'au moins une valeur de réserves fusionnelles sur un moyen d'affichage (930).

11. Dispositif (910) de détermination d'au moins une valeur de réserves fusionnelles d'un sujet, le dispositif (910) étant configuré pour commander une tête de réfracteur automatique (920) et pour :
- commander la tête de réfracteur automatique (920) pour placer, dans un premier emplacement de la tête de réfracteur automatique (920), un premier prisme associé à une première valeur de déviation prismatique,
- commander la tête de réfracteur automatique (920) pour placer, dans un deuxième emplacement de la tête de réfracteur automatique (920), un deuxième prisme associé à une deuxième valeur de déviation prismatique,
- positionner la tête de réfracteur automatique (920) devant une tête du sujet de telle manière que le premier prisme soit positionné en face d'un premier œil du sujet et que le deuxième prisme soit positionné en face d'un deuxième œil du sujet,
- positionner un optotype devant les yeux du sujet,
- commander la tête de réfracteur automatique (920) de sorte à faire défiler automatiquement, de manière croissante, la première valeur de déviation prismatique du premier prisme et la deuxième valeur de déviation prismatique du deuxième prisme, la première valeur de déviation prismatique du premier prisme étant égale à la deuxième valeur de déviation prismatique du deuxième prisme,
- réceptionner une première indication associée à une vision double de l'optotype par le sujet,
- déterminer une valeur de vision double correspondant à la première valeur de déviation prismatique du premier prisme et à la deuxième valeur de déviation prismatique du deuxième prisme à la réception de la première indication,
- commander la tête de réfracteur automatique (920) de sorte à mettre en œuvre un défilement supplémentaire, de manière croissante et à partir de la valeur de vision double, de la première valeur de déviation prismatique du premier prisme et de la deuxième valeur de déviation prismatique du deuxième prisme, la première valeur de déviation prismatique du premier prisme et la deuxième valeur de déviation prismatique du deuxième prisme étant augmentées d'une valeur de déviation prismatique prédéterminée,
- suite à la détermination de la valeur de vision double, commander la tête de réfracteur automatique (920) de manière à faire défiler automatiquement, de manière décroissante, la première valeur de déviation prismatique du premier prisme et de la deuxième valeur de déviation prismatique du deuxième prisme, la première valeur de déviation prismatique du premier prisme étant égale à la deuxième valeur de déviation prismatique du deuxième prisme,
- réceptionner une deuxième indication associée à une vision simple et nette de l'optotype par le sujet,
- déterminer une valeur de recouvrement correspondant à la première valeur de déviation prismatique du premier prisme et à la deuxième valeur de déviation prismatique du deuxième prisme à la réception de la deuxième indication, et
- déterminer au moins une valeur de réserves fusionnelles à partir de la valeur de vision double et de la valeur de recouvrement.

12. Système de détermination d'au moins une valeur de réserves fusionnelles d'un sujet comprenant un dispositif (910) de détermination d'au moins une valeur de réserves fusionnelles selon la revendication 11, une tête de réfracteur automatique (920) et un écran de sélection (930).

13. Produit programme informatique comprenant des instructions exécutables par un processeur (912) et conçues pour mettre en œuvre un procédé selon l'une quelconque des revendications 1 à 10 lorsque que ces instructions sont exécutées par le processeur (912).
